# EUROPEAN PATENT SPECIFICATION

(11) **EP 1 372 764 B1**
(45) Date of publication and mention of the grant of the patent: **09.11.2005**
(21) Application number: 02714371.8
(22) Date of filing: 22.03.2002
(51) Int. Cl.: A61M 5/142

(54) **A LIQUID DISPLACEMENT ARRANGEMENT**
EINE FLÜSSIGKEITSVERDRÄNGUNGSANORDNUNG
DISPOSITIF DE DEPLACEMENT DE LIQUIDE

(30) Priority: 26.03.2001 ZA 200102455
(43) Date of publication of application: 02.01.2004
(73) Proprietor: Wisniewski, Pawel, 4001 Durban (ZA)
(72) Inventor: Wisniewski, Pawel, 4001 Durban (ZA)
(74) Representative: Galgut, John
(86) International application number: PCT/IB2002/000850
(87) International publication number: WO 2002/076532

(56) References cited:
- WO-A-96/34638
- WO-A-99/30755
- DE-C- 4 319 345
- US-A- 4 210 173
- US-A- 4 935 009

## Description

THIS INVENTION relates to a liquid displacement arrangement for displacing a liquid in a controlled manner to the body of a person.

It is required in relation to various medical procedures to displace a liquid in a controlled manner to the body of a person as a part of the procedures. One apparatus for so doing is disclosed in US 4,935,009 wherein this displacement of liquid is manually controlled by using a syringe-type liquid displacement pump, often by an assistant in response to Instructions of the person performing the procedure. Although the person performing the procedure thus has both hands available to perform the procedure, to control the manual actions of an assistant through verbal instructions can be problematic and cause difficulties within procedures. It is thus an object of this invention to at least alleviate the above problem.

The use of a foot operated pump for use with a breast pump is described in WO 96/34638. This arrangement permits milk to be drawn from the user's breast and to be deflected form the suction pipe of the foot operated pump and to be collected in milk bottles. No means are described for the pump to positively drive liquid forwardly for example into the human body.

Although reference is made herein particularly to the displacement of a liquid to the body of a person, procedures requiring similar displacement to the body of an animal also exist. As such, any reference herein to the displacement of a liquid to the body of a person must be interpreted as a reference also to the displacement of a liquid to the body of an animal. It must be understood also that references to displacement of a liquid to the body of a person must be interpreted to include references to the displacement of a liquid into the body of a person, typically via a liquid feed means such as a needle, or the like. Whether a liquid is to be displaced into the body of a person or to the body of a person clearly will be determined in practice by the procedure being performed.

According to the invention there is provided a liquid displacement arrangement for displacing a liquid in a controlled manner to the body of a person, which comprises
a syringe-type pump including a cylinder, a piston displaceable within the cylinder and a piston rod extending from the piston and projecting from the cylinder to enable displacement of the piston and thereby operation of the pump;
an inflow tube of which one end is connected in liquid communication with the cylinder and the opposite free end is connectable to a liquid supply, the inflow tube having a unidirectional valve located in line therewith that permits only flow towards the cylinder, during operation of the pump;
an outflow tube of which one end is connected in liquid communication with the cylinder and the opposite free end is connectable to a liquid feed means for feeding a liquid to the body of a person, the outflow tube having a unidirectional valve located in line therewith that permits only flow from the cylinder, during operation of the pump,
and in which the piston rod and the cylinder of the syringe-type pump each define a locating formation for removably locating the pump between a base member and a foot pedal of a foot operated operating mechanism in a configuration in which displacement of the foot pedal provides for required displacement of the piston of the pump for the operation of the pump.

The liquid displacement arrangement may include a connector formation which communicates with the cylinder of the syringe-type pump and to which the inflow tube and the outflow tube are connected for providing their required liquid communication with the cylinder.

The free end of the inflow tube of the liquid displacement arrangement may be connectable to a liquid supply container while permitting the container to be positioned at different height levels with respect to the body of a person to which a liquid from the liquid supply container must be displaced, in use of the liquid displacement arrangement, and, as such, the unidirectional valves located in line with the inflow tube and the outflow tube respectively, may permit liquid flow in the permitted direction when the hydrostatic pressure acting on the valves, being determined by the position of a liquid supply container to which the inflow tube is connected, in use, exceeds a predetermined pressure level.

The liquid displacement arrangement of the invention may also be provided with a foot operated operating mechanism, for operating the syringe-type pump, the operating mechanism comprising
a base member positionable on a floor surface; and
a foot pedal displaceably mounted on the base member,
the base member and the foot pedal each defining a locating formation complementary to one of the locating formations defined respectively by the piston rod and the cylinder of the syringe-type pump, permitting the removable location of the pump between the base member and the foot pedal in a configuration in which displacement of the foot pedal with respect to the base member provides for operation of the pump.

Particularly for use with a liquid displacement arrangement including such a foot operated operating mechanism, the locating formations defined respectively by the piston rod and the cylinder of the syringe-type pump may be located at the operative remote ends of the piston rod and the cylinder.

The foot pedal of the foot operated operating mechanism may be pivotally mounted on the base member and the locating formations may permit relative pivotal movement respectively between the syringe-type pump and the base member and the syringe-type pump and the foot pedal, the relative pivotal movement permitting axial displacement of the piston of the syringe-type pump by the displacement of the foot pedal.

The foot pedal may be displaceable between a retracted position and a depressed position, which positions respectively coincide substantially with limit positions of the piston of the syringe-type pump within its cylinder. As such, in use of the liquid displacement arrangement, displacement of the pedal from its retracted position to its depressed position may provide for displacement of liquid from the cylinder of the syringe-type pump, whereas displacement of the foot pedal from its depressed position to its retracted position may provide for displacement of liquid into the cylinder.

The foot operated operating mechanism also may include urging means acting between the base member and the foot pedal for urging the foot pedal into its retracted position. Still further, the foot operated foot operating mechanism may include a stop arrangement that acts between the foot pedal and the base member for determining the fully retracted position and the fully depressed position of the pedal with respect to the base member.

The syringe-type pump of the liquid displacement arrangement of the invention may include urging means acting on the piston rod for urging the piston rod into its fully extended position with respect to the cylinder.

A preferred embodiment of the liquid displacement arrangement of the invention provides for the syringe-type pump, the inflow tube and the outflow tube to be provided as a disposable unit of which the pump, in use, is removably located between a base member and a foot pedal of a foot operated operating mechanism.

Further features of the invention, including the mode of use thereof and the benefits associated with the use thereof, are described hereafter with reference to an example illustrated by way of the accompanying diagrammatic drawings. In the drawings:
Figure 1 shows a schematic view of a liquid displacement arrangement, in accordance with the invention; and
Figure 2 shows a detailed schematic side view of the main part of the liquid displacement arrangement of Figure 1.

Referring to the drawings, a liquid displacement arrangement, in accordance with the this invention, is designated generally by the reference numeral 10. The arrangement 10 includes a liquid displacement means, generally designated by the reference numeral 12, and an operating mechanism, generally designated by the numeral 14, for operating the liquid displacement means 12.

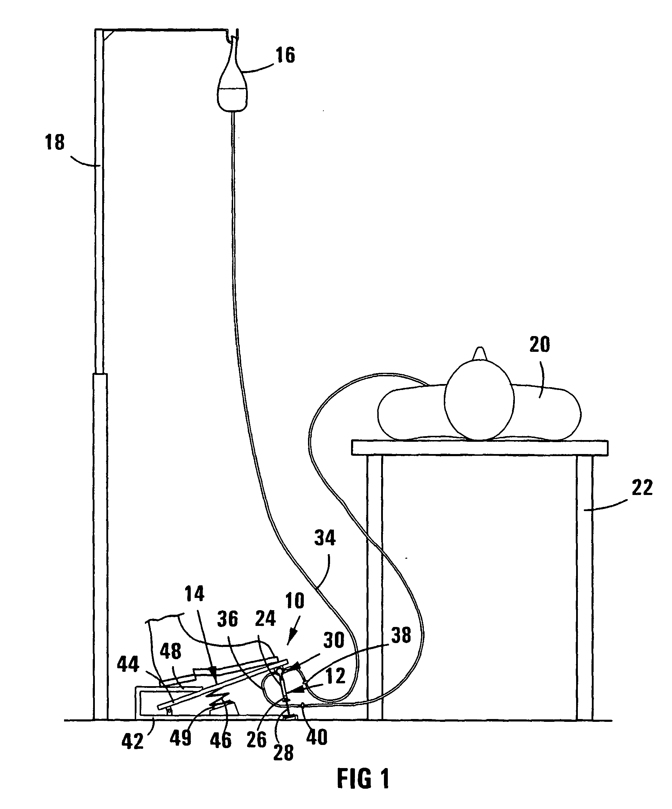

The liquid displacement arrangement is adapted particularly for displacing a liquid in a controlled manner to the body of a person, typically from a liquid supply sachet 16 which is suspended at an elevated level as shown by a support stand 18 which permits the level of the sachet 16 to be adjusted, as is described in more detail hereafter. Figure 1 illustrates also the typical location of the body 20 of a patient lying on a bed structure 22 and requiring a medical procedure that will involve the feed of liquid from the sachet 16 to his body.

The liquid displacement means 12 particularly comprises a syringe-type pump including a cylinder 24, a piston 26 displaceable within the cylinder and a piston rod 28 extending from the piston 26 and projecting from the cylinder 24, the piston rod permitting displacement of the piston 26 within the cylinder 24. The end 30 of the cylinder 24 terminates in a connector formation 32 that it is in liquid communication with the remainder of the cylinder 24, the connector formation 32 having an inflow tube 34 and an outflow tube 36 connected in liquid communication therewith, as shown.

The inflow tube 34, in use of the liquid displacement arrangement 10, is connected to the liquid supply sachet 16 as shown and has a unidirectional valve 38 located in line therewith which permits only flow from the sachet 16 to the cylinder 24. The outflow tube 36 also has a unidirectional valve 40 located in line therewith, the valve 40 permitting flow only from the cylinder 24 to the body 20 of a patient, typically via a liquid feed means (not shown) to which the free end of the outflow tube 36 is connected and which permits required feed of liquid to the body 20.

The operating mechanism 14 includes a base member 42 which has a foot pedal 44 pivotally mounted thereon. A spring 46 acts between the base member and the foot pedal for urging the pedal into its fully retracted position as shown.

Furthermore, a stop arrangement, including a first stop formation 48 and a second stop formation 50, is provided to define respectively the fully retracted position and the fully depressed position of the pedal 44 with respect to the base member 42, these positions coinciding essentially with the limit position of the piston 26 in the cylinder 24 of the syringe-type pump.

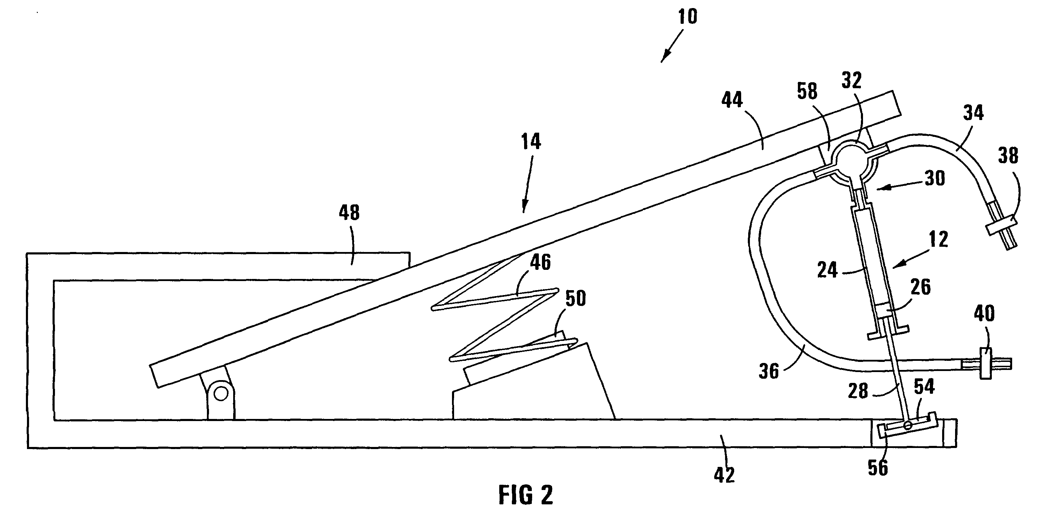

As is shown in detail in Figure 2 of the drawings, the end of the piston rod 28 of the liquid displacement means 12 has a locating formation 54 that cooperates with a complementary locating formation 56 that is pivotally located with respect to the base member 42, providing for releasable pivotal location of this end of the piston rod 28 with respect to the base member 42. The connector formation 32 located at the end 30 of the cylinder 24 itself defines a locating formation that is pivotally located within a complementary locating formation 58 provided near the free end of the foot pedal 44, particularly in the configuration as shown and thereby permitting also relative pivotal displacement between the cylinder 24 and the foot pedal 44. Being releasable from the base member 42 and the foot pedal 44, the liquid displacement means 12 is easily replaceable, whereas the pivotal displacement as described permits the displacement of the piston 26 within the cylinder 24 by the displacement of the foot pedal 44 with respect to the base member 42, without undue sideways forces acting on the piston.

It will be understood that the liquid displacement arrangement 10 will permit, through foot operation, the displacement of liquid from the sachet 16 to the body 20 of a patient, displacement of the foot pedal 44 from its position as shown towards a depressed position providing for displacement of liquid from the cylinder 24 to the body 20 of the patient, whereas reverse displacement of the foot pedal 44 will provide for displacement of further liquid from the sachet 16 into the cylinder 24.

The unidirectional valves 38 and 40 particularly may be configured also to permit a permanent flow of liquid through the flow line defined, with the flow rate being determined by the hydrostatic pressure acting on the valves, which in turn will be determined by the level at which the sachet 16 is suspended. By permitting adjustability of this level, different continuous flow rates can be provided for and particularly by suspending the sachet 16 at a level beneath the body 20 of a patient, continuous flow will cease.

It is envisaged that the syringe-type pump may be of a conventional syringe-type construction, whereas the inflow tube 34 and the outflow tube 36 may be of polyvinyl chloride, latex rubber, or the like. It is envisaged in this regard that the entire liquid displacement means 12 will be disposable, the releasability of this means from the operating mechanism 14 facilitating disposal and the use of a fresh liquid displacement means in association with each different patient and/or in association with each different liquid utilized.

The Applicant believes that the liquid displacement arrangement of the invention is particularly suitable for use in procedures associated with uretroscopy and with the manipulation or destruction of ureteric and kidney stones, which involves an elongate scope to enter the ureteric passage. Liquid can then be displaced into the passage for releasing stones, which will in turn permit gripping of stones to be retracted from the said passage. Insofar as the person performing the procedure himself can now operate the liquid displacement arrangement and, particularly, the rate of liquid displacement, control over liquid displacement is facilitated and known problems associated with these procedures alleviated. The stop formation 48 serves in this regard also to optimize the position of the foot of a person, performing a procedure, with respect to the foot pedal 44 (see Figure 1), i.e. the foot position that will provide maximum control over the operation of the syringe-type pump during the performance of a procedure.

By providing a spring (not shown) to act on the piston rod of the liquid feed means 12 for urging the rod into its extended position as shown, the liquid feed means 12 also can be manually used when separated from the operating mechanism 14, whenever this may be required.

The operating mechanism 14 itself may be made of various different materials , e.g. stainless steel, with the locating formations 56 and 58 thereof being adapted particularly to accommodate the liquid feed means 12, in the configuration described.

## Claims

1. A liquid displacement arrangement (10) for displacing a liquid in a controlled manner to the body of a person (20), which comprises
a syringe-type pump (12) including a cylinder (24), a piston (26) displaceable within the cylinder (24) and a piston rod (28) extending from the piston (26) and projecting from the cylinder (24) to enable displacement of the piston (26) and thereby operation of the pump;
an inflow tube (34) of which one end is connected in liquid communication with the cylinder (24) and the opposite free end is connectable to a liquid supply (16), the inflow tube (34) having a unidirectional valve (38) located in line therewith that permits only flow towards the cylinder (24), during operation of the pump (12);
an outflow tube (36) of which one end is connected in liquid communication with the cylinder (24) and the opposite free end is connectable to a liquid feed means for feeding a liquid to the body of a person (20), the outflow tube (36) having a unidirectional valve (40) located in line therewith that permits only flow from the cylinder (24), during operation of the pump,
**characterised in that** the piston rod (28) and the cylinder (24) of the syringe-type pump (12) each define a locating formation (54 and 32) for removably locating the pump (12) between a base member (42) and a foot pedal (44) of a foot operated operating mechanism (14) in a configuration in which displacement of the foot pedal (44) provides for required displacement of the piston (26) of the pump (12) for the operation of the pump (12).

2. A liquid displacement arrangement as claimed in Claim 1. **characterised by** a connector formation (32) which communicates with the cylinder (24) of the syringe-type pump (12) and to which the inflow tube (34) and the outflow tube (36) are connected for providing their required liquid communication with the cylinder (24).

3. A liquid displacement arrangement as claimed in Claim 1 or Claim 2, **characterised in that** the free end of the inflow tube (34) is connectable to a liquid supply container (16) and permits the container (16) to be positioned at different height levels with respect to the body of a person (20) to which a liquid from the liquid supply container (16) must be displaced, in use of the liquid displacement arrangement, and **in that** the unidirectional valves (38, 40) located in line with the inflow tube (34) and the outflow tube (36) respectively, permit liquid flow in the permitted direction when the hydrostatic pressure acting on the valves, being determined by the position of a liquid supply container (16) to which the inflow tube (34) is connected, in use. exceeds a predetermined pressure level.

4. A liquid displacement arrangement as claimed in any one of Claims 1 to 3, **characterised by** a foot operated operating mechanism (14) for operating the syringe-type pump, the operating mechanism comprising
a base member (42) positionable on a floor surface; and
a foot pedal (44) displaceably mounted on the base member (42),
the base member (42) and the foot pedal (44) each defining a locating formation complementary to one of the locating formations (54 and 32) defined respectively by the piston rod (28) and the cylinder (24) of the syringe-type pump, permitting the removable location of the pump (12) between the base member (42) and the foot pedal (44) in a configuration in which displacement of the foot pedal (44) with respect to the base member (42) provides for operation of the pump (12).

5. A liquid displacement arrangement as claimed in Claim 4, **characterised in that** the locating formations (54 and 32) defined respectively by the piston rod (28) and the cylinder (24) of the syringe-type pump (12) are located at the operative remote ends of the piston rod (28) and the cylinder (24).

6. A liquid displacement arrangement as claimed in Claim 4 or Claim 5, **characterised in that** the foot pedal (44) is pivotally mounted on the base member (42) and the locating formations permit relative pivotal movement respectively between the syringe-type pump (12) and the base member (42) and the syringe-type pump (12) and the foot pedal (44), the relative pivotal movement permitting axial displacement of the piston (26) of the syringe-type pump (12) by the displacement of the foot pedal (44).

7. A liquid displacement arrangement as claimed in any one of Claims 4 to 6, **characterised in that** the foot pedal (44) is displaceable between a retracted position and a depressed position, which positions coincide respectively with limit positions of the piston (26) of the syringe-type pump (12) within its cylinder (24).

8. A liquid displacement arrangement as claimed in any one of Claims 1 to 7 **characterised in that** a connector formation (32) is provided at the end of the cylinder (24) of the pump (12), and **in that** the inflow tube (34), the cylinder (24) and outflow tube (36) are all connected to the connector formation.

9. A liquid displacement arrangement as claimed in Claim 8 **characterised in that** the liquid supply comprises a sachet (16) which is free to be raised and lowered relative to the location of the body of the patent (20).

10. A liquid displacement arrangement as claimed in Claim 9 **characterised in that** the valves (38) and (40) are configured to permit permanent flow of liquid from the sachet (16) to the body of the patient (20).

## Patentansprüche

1. Eine Flüssigkeits-Übertragungsvorrichtung (10) um eine Flüssigkeit in einer kontrollierten Art und Weise zu dem Körper (20) einer Person zuzuführen, bestehend aus
einer spritzenartigen Pumpe (12) bestehend aus einem Zylinder (24), einem innerhalb des Zylinders (24) verschiebbaren Kolben (26) und einer Kolbenstange, die sich von dem Kolben (26) erstreckt und aus dem Zylinder (24) herausragt um die Verschiebung des Kolbens (26) und **dadurch** den Betrieb der Pumpe zu ermöglichen;
einem Zulaufschlauch (34), von dem das eine Ende einen Flüssigkeitsaustausch mit dem Zylinder (24) herstellt und das gegenüberliegende freie Ende anschließbar ist an eine Flüssigkeitsversorgung(16); der Zulaufschlauch (34) ist mit einem unidirektionalen Ventil (38) in Durchflussrichtung versehen, in der Art und Weise, dass während der Betätigung der Pumpe (12) eine Fließbewegung nur in Richtung auf den Zylinder (24) möglich ist;
einem Ablaufschlauch (36), von dem das eine Ende einen Flüssigkeitsaustausch mit dem Zylinder (24) herstellt und das gegenüberliegende freie Ende anschließbar ist an ein Flüssigkeit übertragendes Element, um eine Flüssigkeit dem Körper eines Menschen (20) zuzuführen; der Ablaufschlauch (36) ist mit einem unidirektionalen Ventil (40) in Durchflussrichtung versehen, in der Art und Weise, dass während der Betätigung der Pumpe (12) eine Fließbewegung nur von dem Zylinder möglich ist,
**gekennzeichnet dadurch, dass** die Kolbenstange (28) und der Zylinder (24) der spritzenartigen Pumpe (12) je ein Positionierungsgebilde (54 und 32) definieren, um die Pumpe (12) entfernbar zwischen einem Bodenelement (42) und einem Fußpedal (44) eines fußbetätigten Bedienungsmechanismus (14) zu positionieren - in einer Konfiguration, in der die Verlagerung des Fußpedals (44) die erforderliche Verlagerung des Kolbens der Pumpe (12) zur Funktion der Pumpe bewirkt.

2. Eine Flüssigkeits-Übertragungsvorrichtung nach Anspruch 1, mag ein Verbindungselement (32) enthalten, welches mit dem Zylinder (24) der spritzenartigen Pumpe (12) in Verbindung steht und zu dem der Zulaufschlauch (34) und der Ablaufschlauch (36) angeschlossen sind, um für die jeweilig erforderliche Flüssigkeitsübertragung mit dem Zylinder (24) zu sorgen.

3. Eine Flüssigkeits-Übertragungsvorrichtung nach Anspruch 1 oder Anspruch 2, **gekennzeichnet dadurch, dass** das freie Ende des Zulaufschlauches (34) in einer Weise an einen Flüssigkeitsvorratsbehälter (16) anschließbar ist, die die Positionierung des Behälters in verschiedenen Hlöhenniveaus in Relation zu dem Körper einer Person (20), zu der mittels Verwendung der Flüssigkeits-Übertragungsvorrichtung eine Flüssigkeit aus dem Flüssigkeitsvorratsbehälter (16) zugeführt werden soll, ermöglicht,
und **dadurch**, dass die jeweils in Durchflussrichtung mit dem Zulauf- und Ablaufschlauch angeordneten unidirektionalen Ventile eine Flüssigkeitsbewegung in die zugelassene Richtung ermöglichen, wenn der auf die Ventile wirkende hydrostatische Druck, welcher bestimmt wird durch die Positionierung eines Flüssigkeitsvorratsbehälters (16), zu dem der sich in Betrieb befindliche Zulaufschlauch (34) verbunden ist, den vorgegebenen Druck überschreitet.

4. Eine Flüssigkeits-Übertragungsvorrichtung nach Anspruch 1, 2 oder 3, **gekennzeichnet durch** einen mit dem Fuß bedienbaren Bedienungsmechanismus (14) um die spritzenartige Pumpe zu betätigen; der Bedienungsmechanismus umfasst
ein auf dem Fußboden positionierbares Bedenelement (42); und
ein auf dem Bodenelement (42) beweglich montiertes Fußpedal (44),
das Bedenelement (42) und das Fußpedal beschreiben jeweils eine Positionsanordnung, die abgestimmt ist auf einer der Positionierung (54 und 32), die entsprechend **durch** die Kolbenstange (28) und den Zylinder (24) der spritzenartigen Pumpe vorgegeben ist; diese Positionierung ermöglicht die entfembare Platzierung der Pumpe (12) zwischen dem Bodenelement (42) und dem Fußpedal (44) in einer Konfiguration, in der die Verlagerung des Fußpedals (44) im Verhältnis zu dem Bodenelement (42) für die Funktion der Pumpe (12) sorgt.

5. Eine Flüssigkeits-Übertragungsvorrichtung nach Anspruch 4, **gekennzeichnet dadurch, dass** die Positionierungen (54 und 32), die jeweils durch die Kolbenstange (28) und den Zylinder (24) der spritzenartigen Pumpe (12) definiert sind, sich am jeweiligen Ende es Arbeitsbereichs der Kolbenstange (28) und des Zylinders (24) befinden.

6. Eine Flüssigkeits-Übertragungsvorrichtung nach Anspruch 4 oder Anspruch 5, **gekennzeichnet dadurch, dass** das Fußpedal (44) schwenkbar auf das Bodenelement (42) montiert ist und die Positionierungen eine relativ schwenkbare Bewegung jeweils zwischen der spritzenartigen Pumpe (12) und dem Bodenelement (42) sowie zwischen der spritzenartigen Pumpe (12) und dem Fußpedal (44) ermöglichen, wobei die relativ schwenkbare Bewegung eine axiale Verlagerung des Kolbens (26) der spritzenartigen Pumpe durch die Verlagerung des Fusspedals ermöglicht.

7. Eine Flüssigkeits-Übertragungsvorrichtung nach jedem der Ansprüche 4 bis 6, **gekennzeichnet dadurch, dass** das Fußpedal beweglich ist zwischen einer entspannten Position zu einer heruntergedrückten Position; die Positionen stimmen im Wesentlichen überein mit der jeweiligen Raumbegrenzung des Kolbens (26) innerhalb des Zylinders (24) der spritzenartigen Pumpe (12).

8. Eine Flüssigkeits-Übertragungsvorrichtung nach jedem der Ansprüche 1 bis 7, **gekennzeichnet dadurch, dass** sich ein Verbindungselement (32) am Ende des Zylinders (24) der Pumpe (12) und **dadurch**, dass der Zulaufschlauch (34), der Zylinder (24) und der Ablaufschlauch (36) alle and das Verbindungselement angeschlossen sind.

9. Eine Flüssigkeits-Übertragungsvorrichtung nach Anspruch 8, **gekennzeichnet dadurch, dass** der Flüssigkeitsvorrat aus einem Beutel (16) besteht, der im Verhältnis zum Körper des Patienten (20) frei beweglich erhöht oder gesenkt werden kann.

10. Eine Flüssigkeits-Übertragungsvorrichtung nach Anspruch 9, **gekennzeichnet dadurch, dass** die Ventile (38) und (40) gestaltet sind, um eine permanente Fließbewegung einer Flüssigkeit von dem Beutel (16) zu dem Körper eines Patienten (20) zuzulassen.

## Revendications

1. Un système de transport de liquide (10) pour transporter un liquide de manière contrôlée au corps d'une personne (20), qui comprend:
une pompe type-seringue (12) comprenant un cylindre (24), un piston (26) mobile dans le cylindre (24) et une tige de piston (28) s'étendant du piston (26) et se projetant du cylindre (24) afin de permettre le mouvement du piston (26) et donc celui de l'opération de la pompe;
un tube d'arrivée (34) dont une extrémité est connectée dans un flux de liquide avec le cylindre (24) et l'extrémité libre opposée est connectée à une alimentation de liquide (16), le tube d'arrivée (34) est muni d'une valve unidirectionelle (38) située dans la ligne qui permet la circulation uniquement vers le cylindre (24), pendant l'opération de la pompe (12);
un tube de sortie (36) dont une extrémité est connectée dans une flux de liquide avec le cylindre (24) et l'extrémité libre opposée peut être connectée à un système d'alimentation de liquide pour alimenter un liquide au corps d'une personne (20), le tube de sortie (36) ayant une valve unidirectionnelle (40) située dans la ligne qui permet uniquement une circulation à partir du cylindre (24), pendant l'opération de la pompe, qui est **caractérisée par le fait que** la tige de piston (28) et le cylindre (24) de la pompe type-seringue (12) chacun définit un point de localisation (54 et 32) pour localiser de façon détachable la pompe (12) entre le membre de base (42) et la pédale (44) d'un mécanisme opéré par le pied (14) en une configuration dans laquelle le déplacement de la pédale (44) pourvoit le déplacement nécessaire du piston (26) de la pompe (12) pour l'opération de la pompe (12).

2. Un système de transport de liquide comme il est établi dans la revendication 1 **caractérisé par** une disposition de connexion (32) qui communique avec le cylindre (24) de la pompe type-seringue (12) et à laquelle le tube d'arrivée (34) et le tube de sortie (36) sont connectés afin de pourvoir leur flux de liquide nécessaire avec le cylindre (24)

3. Un système de transport de liquide comme il est établi dans la revendication 1 ou 2, **caractérisé par le fait que** l'extrémité libre du tube d'arrivée (34) peut être connectée à un sac contenant l'alimentation de liquide (16) et qui permet que le sac (16) soit positionné à différentes hauteurs relativement au corps de la personne (20) à qui un liquide du sac d'alimentation (16) doit être administré, en utilisant le système de transport de liquide, et que les valves unidirectionnelles (38, 40) situées dans la ligne d'acheminement dans le tube d'arrivée (34) et le tube de sortie (36) respectivement, permettent la circulation de liquide dans la direction voulue quand la pression hydrostatique s'appliquant sur les valves, étant déterminée par la position du sac d'alimentation de liquide (16) avec quoi le tube d'arrivée (34) est connecté, en usage, dépasse un niveau de pression prédéterminé.

4. Un système de transport de liquide comme il est établi dans la déclaration 1 à 3, **caractérisé par** un mécanisme d'opération à pédale (14) pour opérer la pompe type-seringue, le mécanisme d'opération comprenant:
un membre de base (42) qui peut être placé sur le sol; et
une pédale (44) montée de façon amovible sur le membre de base,
le membre de base (42) ainsi que la pédale (44) chacun définissant une disposition de localisation complémentaire à un des points de localisation (54 et 32) définis respectivement par la tige du piston (28) et le cylindre (24) de la pompe type-seringue, permettant la position amovible de la pompe (12) entre le membre de base (42) et la pédale (44) en une configuration dans laquelle le déplacement de la pédale (44) relatif au membre de base (42) déclenche l'opération de la pompe (12).

5. Un système de transport de liquide comme il est établi dans la revendication 4, **caractérisé par le fait que** les dispositions de localisation (54 et 32) définies respectivement par la tige de piston (28) et le cylindre (24) de la pompe type-seringue (12) sont situés aux extrémités opératives de la tige du piston (28) et le cylindre (24).

6. Un système de transport de liquide comme il est établi dans la revendication 4 ou 5, **caractérisé par le fait que** la pédale (44) est montée centralement sur le membre de base (42) et les dispositions de localisations permettent un mouvement central relatif respectivement entre la pompe type-seringue (12) et le membre de base (42) et la pompe type-seringue (12) et la pédale (44), et le mouvement central relatif permettant le déplacement axial du piston (26) de la pompe type-seringue (12) par le déplacement de la pédale (44).

7. Un système de transport de liquide comme il est établi dans aucune des revendications 4 à 6, **caractérisé par le fait que** la pédale est déplaçable entre la position rétractée et la position abaissée, lesquelles positions coincident respectivement avec les positions limites du piston (26) de la pompe type seringue (12) dans le cylindre (24).

8. Un système de transport de liquide comme il est établi dans les revendications 1 à 7 **caractérisé par le fait qu'**un point de connexion (32) est pourvu à l'extrémité du cylindre (24) de la pompe (12), et que le tube d'arrivée (34), le cylindre (24) et le tube de sortie (36) sont tous connectés à ce point de connexion.

9. Un système de transport de liquide comme il est établi dans la revendication 8 **caractérisé par le fait que** l'alimentation de liquide comprend un sachet (16) qui peut être élevé ou abaissé librement suivant la position du corps du patient (20).

10. Un système de transport de liquide comme il est établi dans la revendication 9 **caractérisé par le fait que** les valves (38) et (40) sont conçues pour permettre la circulation permanente du liquide du sachet (16) au corps du patient (20).
